Europäisches Patentamt

(19) European Patent Office

Office européen des brevets

(11) Publication number: **0 082 345**
**B1**

(12) **EUROPEAN PATENT SPECIFICATION**

(45) Date of publication of patent specification: **13.08.86**

(51) Int. Cl.⁴: **A 61 M 1/36**

(21) Application number: **82110914.7**

(22) Date of filing: **25.11.82**

(54) Apparatus for therapeutic immunodepletion.

<table>
<tr><td>

(30) Priority: **30.11.81 US 326134**

(43) Date of publication of application:
**29.06.83 Bulletin 83/26**

(45) Publication of the grant of the patent:
**13.08.86 Bulletin 86/33**

(84) Designated Contracting States:
**DE FR GB SE**

(56) References cited:
**WO-A-80/02805**
**GB-A-1 491 261**
**US-A-3 228 876**
**US-A-4 031 201**
**US-A-4 061 141**
**US-A-4 215 688**
**US-A-4 252 653**

</td><td>

(73) Proprietor: **Cordis Corporation**
**10555 West Flagler Street**
**Miami, Florida 33174 (US)**

(72) Inventor: **Halbert, Seymour P.**
**12450 Rock Garden Lane**
**Miami Florida 33156 (US)**

(74) Representative: **Blumbach Weser Bergen**
**Kramer Zwirner Hoffmann Patentanwälte**
**Radeckestrasse 43**
**D-8000 München 60 (DE)**

</td></tr>
</table>

Courier Press, Leamington Spa, England.

## Description

Numerous illnesses are caused by excessive levels of harmful agents in the blood stream. Two large classes of such disturbances include those which are associated with:

1) Substances in toxic excess concentrations, such as externally derived drugs, e.g. digoxin, gentamicin, etc. and internally derived substances, e.g. thyroxin in thyrotoxicosis, etc.; or
2) Abnormal auto-antibodies which are directed against normal tissue constituents, such as rheumatoid factor, anti-DNA, etc.

In some of these disturbances, particularly the latter group, recent studies have indicated that plasmapheresis exerts a beneficial therapeutic effect, apparently as a cleansing mechanism. However, plasmapheresis is a relatively crude method of removing unwanted deleterious substances, since it results in the depletion of virtually all plasma constituents, including many essential beneficial ones, in addition to the removal of the harmful agent. Ideally, a highly selective system is required which removes *only* the unwanted harmful agent, and leaves all the other plasma constituents unchanged in the patient. The resultant therapeutic effects would presumably be considerably more efficient and thorough, with no side effects due to the treatment.

Only one approach with the required high degree of selectivity is presently known. This approach involves immunological reagents (antibodies, antigens or haptens), which have been shown to possess remarkable degrees of specificity. The use of such reagents in solid phase has proven feasible in numerous demonstrations during the last 10 to 20 years. Thus, it has been established in the art that antibodies and antigens can be bound covalently to solid supports in a fully reactive state while retaining complete specificity. This "solid phase" technology is the basis for a large proportion of the sensitive radioimmunoassays and enzyme-immunoassays currently used in diagnostic laboratories.

The prior art literature reveals several suggested therapeutic methodologies employing solid-phase immunoadsorbent columns. Where the immunoadsorbent is adsorbed on a solid phase such as activated charcoal, there exists the possibility of leakage of the immunoadsorbent into the blood which might provoke an immune response within the patient, thus producing undesirable side effects. Even where such prior art columns have employed immunoadsorbents covalently bound to a particulate solid substrate, the column performance in the treatment of blood has proven to be less than entirely satisfactory, for use as a shunt between an artery and vein, because of the tendency of the packed column to become plugged. Another approach suggested in the literature is the use of an extracorporeal shunt in the form of a chamber containing a number of polymethylmethacrylate plates to which a proteinaceous immunoadsorbent is covalently bound. See: Holger Hyden "Enzyme Therapy and Immuno-adsorption by an Extra-Corporeal Device"; *Biomat. Med. Art. Org.,* 1980, 8, (1), pp. 1—11. The limitations imposed by the use of such a device relate to the limited amount of surface areas with bound immunoadsorbent and facility for regeneration.

From the document WO—A—80/02805 a therapeutic apparatus for the removal of a harmful agent from blood is known. This known apparatus includes a bundle of parallel hollow fibers and a header at each end of the bundle connecting said fibers to a flow passage. A coating of an immunoadsorbent for the harmful agent is covalently bound to the respective exterior surfaces of said hollow fibers through a suitable coupling agent. In this prior art whole blood passes through the interior of the hollow fibers while the immunoadsorbent is bound to the respective exterior surfaces of the fibers. A fraction of the whole blood passes through the fiber walls and only this penetrating fraction contacts the biologically active material that is on the exterior surface, i.e. on the surface of the side of the fiber walls that faces away from the whole blood. In this way, a portion of the whole blood does not pass through these fiber walls and thus does not contact the biologically active material. In order to have the whole blood separated, a portion of it treated and the whole blood reassembled this prior art requires the impartation of variable pressure into the device through a connecting nipple in order to assist in achieving the separation and reassembly, which requires passage of the penetrating fraction of the whole blood through the walls of the hollow fibers in both directions.

The document US—A—4,252,653 discloses covalent bonding of immunoadsorbents and various other chemically active substances to fibers. The fibers are not hollow and define a yarn which is wound on the inlet spindle of their blood treatment apparatus to form a fiber coil. The blood is passed over the exterior surface of the yarn.

Ideally any extracorporeal shunt should be of a nature which allows therapeutic treatment to be conducted continuously over as long a period of time as is appropriate to the patient's condition. Accordingly, it is an object of the present invention to provide a therapeutic device having a solid phase immobilized immunoadsorbent specific for a harmful agent to be removed from blood, blood plasma or serum and of a nature which permits continuous, prolonged use as an extracorporeal shunt when connected between an artery and vein of a patient.

Consistent with the aforementioned objective, it is a further objective of the present invention to provide such a device which may be easily and rapidly regenerated without interruption of therapeutic treatment.

It is yet another object of the present invention to provide a relatively large surface area of bound immunoadsorbent relative to the volumetric flow rate of the blood undergoing treatment.

Yet another object of the invention is to provide such a device characterized by minimal pressure drop and susceptibility to plugging.

These objects are achieved with an apparatus as claimed in claim 1.

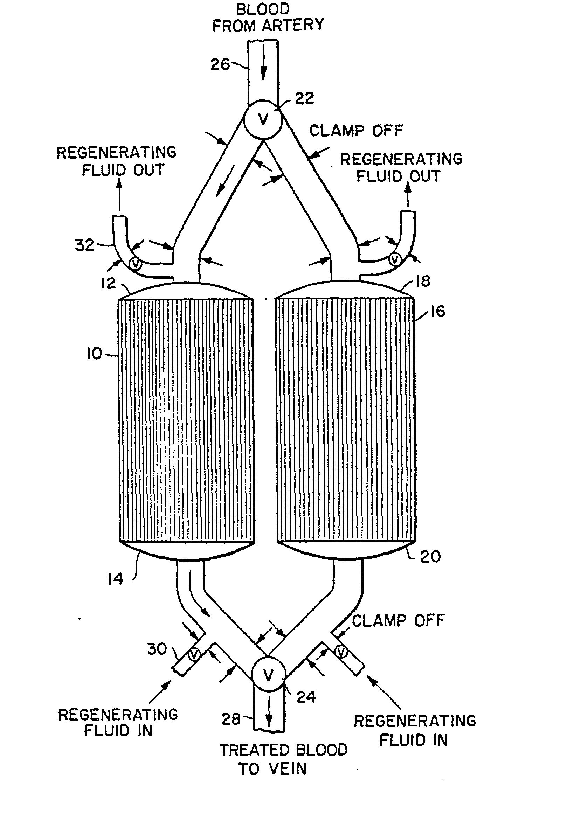

These and other objects and features of the present invention will become apparent to those skilled in the art from a reading of the description which follows in conjunction with the accompanying drawing.

This invention provides a hollow fiber therapeutic device for the removal of a harmful agent from blood, blood plasma or serum. The therapeutic device of the invention includes one or more hollow fiber bundles wherein the interior surface of each hollow fiber is coated with a proteinaceous or other immunoadsorbent covalently bonded to the hollow fiber solid phase. Preferably, a plurality of such hollow fiber bundles are employed in a parallel array so that a patient's blood may be continuously treated by passage through one or more selected bundles retaining immunoadsorbent capacity at a suitable level while other bundles, removed from service, are simultaneously undergoing regeneration. With such an array of parallel bundles the apparatus may be used as an extracorporeal shunt between an artery and a vein of a patient continuously, over any extended period of time suitable for treatment of the patient's condition. Specific examples of proteinaceous immunoadsorbents covalently bound to the fiber interiors in accordance with the present invention are human immunoglobulin G, for the treatment of rheumatoid arthritis and anti-digoxin for the removal of digoxin.

The hollow fiber bundles employed in the present invention are prepared from hollow fiber bundles of the type commercially available for use in an artificial kidney apparatus. Fiber bundles of this type are shown, for example, in US—A—4,211,597 and in US—A—3,228,876 and are commercially available from the Cordis-Dow Corporation. Such hollow fiber bundles contain thousands of small diameter hollow fibers typically composed of cellulose. The individual fibers are mounted within a common chamber wherein their open ends terminate in spaced, subdivided chambers or headers formed in each end of the main chamber. A proteinaceous or other immunoadsorbent specific to the harmful agent to be removed from a patient's blood is covalently bound to the interior cellulose fiber surfaces through a suitable coupling agent. The coupling agent may consist of aldehyde groups formed on the surface of the cellulose by treatment of the cellulose, for example, by sodium periodate. An alternative coupling technique is the introduction of reactive groups into the surface of the cellulose by treatment with a cyanogen bromide solution. In like manner, hollow fibers of polystyrene may be coated with a proteinaceous or other immunoadsorbent through isothiocyanate groups introduced onto the polystyrene surfaces. Yet another alternative is the use of hollow glass fibers having a proteinaceous immunoadsorbent covalently bound thereto through a silane coupling agent as taught, for example, in US—A—3,652,761. Human immunoglobulin G and anti-digoxin and other specific immunoadsorbents may be covalently bound to the lumen surfaces of cellulose hollow fibers through the aforementioned coupling agents.

A hollow fiber bundle preferred for use in the present invention consists of a bundle of at least two hundred hollow cellulose fibers and, more preferably, up to ten thousand such fibers and further having the immunoadsorbent covalently bound to the fiber lumen surfaces through aldehyde or cyanogen bromide activated groups.

The sole drawing figure is a schematic representation of an embodiment of the present invention including two hollow fiber bundles connected in parallel.

The drawing depicts an embodiment of the present invention wherein two hollow fiber bundles, generally designated 10 and 16, are connected in parallel for use as an extracorporeal shunt connected between an artery and vein of a patient to be treated for removal of some harmful agent from the bloodstrem. The drawing shows an array of two fiber bundles, of the type described in US—A—3,228,876, but having a specific immunoadsorbent covalently bound to the lumen surfaces. In actual use as an extracorporeal shunt inlet 26 is connected to an artery of the patient to be treated and outlet 28 is connected to a vein of the patient. Incoming blood from the artery is directed by valve 22, for example, to fiber bundle 10 and valve 24 is likewise positioned to direct the blood exiting fiber bundle 10 through outlet 28 to the patient's vein. The incoming blood enters header 12 wherein it is distributed for flow passage through the individual fibers of bundle 10, which preferably consist of approximately ten thousand parallel hollow cellulose fibers. Blood exiting bundle 10 is collected in header 14 and returned through valve 24 and outlet 28 to the patient's vein. Experimentation with the apparatus may establish a preferred service period for a single bundle and, at the expiration of such a period, the blood flow may be diverted from bundle 10 through valve 22 to header 18 through bundle 16 to be collected in header 20 and returned to the patient's vein through valve 24 and outlet 28. Alternatively, the treated blood may be sampled at 30 to determine when the blood flow should be diverted to the second bundle. After diversion of the blood flow to bundle 16, the blood contained in bundle 10 is thoroughly flushed out with a balanced salt solution. Then a low pH buffer, of approximately pH 3.0 or less, or another antigen-antibody dissociating solution is introduced at 30 through fiber bundler 10, exiting at 32. A preferred low pH buffer is 0.1 molar glycine hydrochloride of pH 2.5. After flushing with balanced salt solution, the unit is ready to be re-used. In this manner, the immunoadsorbent capacity of fiber bundle 10 is restored to its original value. In like manner, fiber bundle 16 may be regenerated after a predetermined period of time or detection of the immunoadsorbent capacity dropping to a predetermined level. An optional alternative to the valve depicted in the drawing is the use of surgical clamps at the points indicated by the double arrows ($\rightarrow \leftarrow$).

In the treatment of rheumatoid arthritis, the goal is the specific removal from the blood stream of the autoantibody (rheumatoid factor) which is considered to be involved in the pathogenesis of the disease. This auto-antibody is specifically directed against a certain conformation of human immunoglobulin G (IgG) acting as antigen. This antigen may be isolated from human plasma and purified to a considerable degree.

This "antigen", human IgG is covalently bound to the lumen surfaces of the aforementioned hollow fiber devices for use in the treatment of rheumatoid arthritis. After a predetermined period of time or after the immunoadsorption capacity of a given hollow fiber unit drops to a predetermined value, that hollow fiber unit is removed from service while the blood is diverted for circulation through an identical alternative unit. While the second unit is in service the first is regenerated for additional use. In the regeneration process, the bundle removed from service is first thoroughly rinsed with a sterile balanced salt solution to remove all the blood and plasma. Subsequently, the rinsed fiber bundle is washed with a buffer at a pH of approximately 2.5 to liberate the antibodies (rheumatoid factors) from their immuno-bond with the "antigen" (human IgG). After washing again with sterile balanced salt solution at neutral pH, the fully reactivated unit will once more be available for use in treating the patient's blood. Each unit is alternatively used in this manner until the patient's plasma is essentially free of rheumatoid factor autoantibodies. In this manner, the unwanted harmful agent may be removed from the patient's blood without effecting any change on the other constituents of the blood.

Digoxin may be removed from a patient's blood in essentially the same manner as described above in conjunction with the treatment of rheumatoid arthritis. However, in the removal of digoxin the antibody against digoxin (anti-digoxin) is covalently bound to the lumen surfaces of the fiber bundles. The anti-digoxin may be prepared in goats and purified immunochemically.

In the systems described in the foregoing, arterial pressure is normally sufficient for circulating the blood through the hollow fiber units.

The invention may be embodied in other specific forms. For example, by choice of specific antigen or antibody covalently bound to the lumen surfaces of the hollow fibers, other units may be prepared for the treatment of various auto-immune disturbances such as systemic lupus erythematosus, myasthenia gravis, glomerulonephritis, auto-immune thyroiditis, etc. Each illness to be treated requires its own specific purified antigen or antibody immobilized in the solid phase, i.e. covalently bound to the lumen surfaces of the fibers. Likewise similar systems may be used to reduce toxic levels of a wide variety of drugs, e.g. the antibiotic gentamicin, the anti-cancer agent methotrexate, the antiasthmatic drug theophylline, the cardiac drugs, lidocaine and procainamide, drugs of abuse, heroin etc. In these cases, the appropriate antibody would be covalently bound to the fiber lumen surfaces for each drug treatment. Thus, the embodiments described above should be considered in all respects as illustrative and not restrictive, the scope of the invention being indicated by the appended claims rather than by the foregoing description.

## Claims

1. A therapeutic apparatus for the removal of a harmful agent from blood, blood plasma or serum, said therapeutic apparatus including two or more bundles (10; 16) of parallel hollow fibers, a separate header (12; 14; 18; 20) at each end of each of said respective bundles connecting said fibers to a separate flow passage at each said respective end for flow of said blood, blood plasma or serum through the respective hollow interiors of said fibers, and a coating of an immunoadsorbent for the harmful agent, the immunoadsorbent being covalently bound to the respective interior surfaces of said hollow fibers through a suitable coupling agent, said separate flow passages at each end of said respective bundles communicate with a respective common flow passage (26; 28) through respective conduits, and means (22; 24) for diverting the flow of blood, blood plasma or serum from one hollow fiber bundle (10; 16) to another hollow fiber bundle (16; 10) and means for selectively introducing a regenerating fluid into said respective conduits and through one or more bundles from which the blood, blood plasma or blood serum flow has been diverted, said selective regenerating fluid introducing means including a closeable introduction conduit (30) and a closeable exit conduit (32) for flowing regenerating fluid through said one or more bundles in a direction countercurrent to the blood flow direction.

2. The apparatus of claim 1, wherein each of said bundles (10, 16) comprises two hundred or more separate hollow fibers.

3. The apparatus of claim 1 or 2, wherein said immunoadsorbent is human immunoglobulin G for the removal of rheumatoid factor.

4. The apparatus of claim 1 or 2, wherein said immunoadsorbent is anti-digoxin for the removal of digoxin.

5. The apparatus of claim 1 or 2, wherein said immunoadsorbent is an antigen.

6. The apparatus of claim 1 or 2, wherein said immunoadsorbent is an antibody.

## Patentansprüche

1. Therapeutische Vorrichtung zur Entfernung eines schädlichen Stoffes aus Blut, Blutplasma oder -serum, umfassend

zwei oder mehr Bündel (10; 16) paralleler hohler Fasern,

einen gesonderten Sammler (12; 14; 18; 20) an jedem Ende jedes Bündels, der die Fasern an dem jeweiligen Ende mit einem gesonderten Strömungskanal verbindet, damit das Blut, Blutplasma oder -serum durch das jeweilige hohle Innere der Fasern strömen kann,

eine Beschichtung aus einem Immunoadsorbens für den schädlichen Stoff, welches über ein geeignetes Kupplungsmittel kovalent an die jeweiligen Innenflächen der hohlen Fasern gebunden ist,

wobei die getrennten Strömungskanäle an den Enden der jeweiligen Bündel über jeweilige Leitungen mit einem jeweiligen gemeinsamen Strömungskanal (26; 28) kommunizieren,

Mittel (22; 24) zum Umlenken des Flusses von Blut, Blutplasma oder -serum von einem hohlen Faserbündel (10; 16) zu einem anderen hohlen Faserbündel (16; 10), und

Mittel zum selektiven Einleiten eines Regenerierfluids in die jeweiligen Leitungen und durch eines oder mehrere Bündel, von denen der Fluß aus Blut, Blutplasma oder Blutserum umgelenkt wurde,

wobei die Mittel zum selektiven Einleiten des Regenerierfluids eine schließbare Zuflußleitung (30) und eine schließbare Abflußleitung (32) aufweisen, damit das Regenerierfluid durch das eine oder die mehreren Bündel in einer Gegenstromrichtung zur Blutstromrichtung fließen kann.

2. Vorrichtung nach Anspruch 1, bei der jedes der Bündel (10, 16) zweihundert oder mehr gesonderte hohle Fasern enthält.

3. Vorrichtung nach Anspruch 1 oder 2, bei der das Immunoadsorbens menschliches Immunoglobulin G für die Entfernung eines rheumatoiden Faktors ist.

4. Vorrichtung nach Anspruch 1 oder 2, bei der das Immunoadsorbents Antidigoxin für die Entfernung von Digoxin ist.

5. Vorrichtung nach Anspruch 1 oder 2, bei der das Immunoadsorbens ein Antigen ist.

6. Vorrichtung nach Anspruch 1 oder 2, bei der das Immunoadsorbens ein Antikörper ist.

## Revendications

1. Appareil thérapeutique pour l'élimination d'un agent nocif du sang, du plasma sanguin ou du sérum, ledit appareil thérapeutique comprenant deux faisceaux ou plus (10; 16) de fibres creuses parallèles, un collecteur séparé (12; 14; 18; 20) à chaque extrémité de chacun desdits faisceaux respectifs raccordant lesdites fibres à un passage d'écoulement séparé à chacune desdites extrémités respectives pour l'écoulement dudit sang, dudit plasma sanguin ou dudit sérum dans les intérieures creux respectifs desdites fibres, et un revêtement d'un immuno-adsorbant pour l'agent nocif, l'immuno-adsorbant étant en liaison de covalence avec les surfaces intérieures respectives desdites fibres creuses par l'intermédiaire d'un agent convenable de couplage, lesdits passages d'écoulement séparés à chaque extrémité desdits faisceaux respectifs communiquent avec un passage d'écoulement commun respectif (26; 28) par l'intermédiaire de conduits respectifs, et des moyens (22; 24) destinés à dévier l'écoulement de sang, de plasma sanguin ou de sérum d'un faisceau (10; 16) de fibres creuses vers un autre faisceau (16; 10) de fibres creuses et des moyens destinés à introduire sélectivement un fluide régénérant dans lesdits conduits respectifs et dans un ou plusieurs faisceaux desquels l'écoulement de sang, de plasma sanguin ou de sérum sanguin a été dévié, lesdits moyens d'introduction d'un fluide régénérant sélectif comprenant un conduit (30) d'introduction pouvant être fermé et un conduit (32) de sortie pouvant être fermé pour l'écoulement d'un fluide régénérant à travers ledit ou lesdits faisceaux, à contre-courant de l'écoulement de sang.

2. Appareil selon la revendication 1, dans lequel chacun desdits faisceaux (10, 16) comprend deux cents, ou plus, fibres creuses séparées.

3. Appareil selon la revendication 1 ou 2, dans lequel ledit immuno-adsorbant est de l'immunoglobuline G humaine pour l'élimination du facteur rhumatoïde.

4. Appareil selon la revendication 1 ou 2, dans lequel ledit immuno-adsorbant est l'anti-digoxine pour l'élimination de la digoxine.

5. Appareil selon la revendication 1 ou 2, dans lequel ledit immuno-adsorbant est un antigène.

6. Appareil selon la revendication 1 ou 2, dans lequel ledit immuno-adsorbant est un anticorps.

0 082 345

BLOOD FROM ARTERY

REGENERATING FLUID OUT

CLAMP OFF

REGENERATING FLUID OUT

REGENERATING FLUID IN

CLAMP OFF

REGENERATING FLUID IN

TREATED BLOOD TO VEIN

1